(19) Europäisches Patentamt
European Patent Office
Office européen des brevets

(11) EP 0 579 020 B1

(12) **EUROPÄISCHE PATENTSCHRIFT**

(45) Veröffentlichungstag und Bekanntmachung des Hinweises auf die Patenterteilung:
**21.01.1998 Patentblatt 1998/04**

(51) Int Cl.$^6$: **C07D 239/54**, C07D 239/30

(21) Anmeldenummer: **93110267.7**

(22) Anmeldetag: **28.06.1993**

(54) **Verfahren zur Herstellung von 5-(Trifluormethyl)-uracil und die neuen Verbindungen 2,4-Dichlor-5-trichlormethylpyrimidin und 2,4-Difluor-5-trifluormethyl-pyrimidin**

Process for the preparation of 5-(Trifluoromethyl) uracil and as new compounds 2,4-dichloro-5-trichloromethyl pyrimidine and 2,4-dufluoro-5-trifluoromethyl-pyrimidine

Procédé de préparation du 5-(Trifluorométhyl) uracile et les nouveaux composés 2,4-dichoro-5-trichlorométhyl pyrimidine et 2,4-difluoro-5 trifluorométhyl-pyrimidine

(84) Benannte Vertragsstaaten:
**BE CH DE FR GB IT LI NL**

(30) Priorität: **09.07.1992 DE 4222518**

(43) Veröffentlichungstag der Anmeldung:
**19.01.1994 Patentblatt 1994/03**

(73) Patentinhaber: **BAYER AG**
**51368 Leverkusen (DE)**

(72) Erfinder:
- **Andres, Peter, Dr.**
  **D-42799 Leichlingen (DE)**
- **Marhold, Albrecht, Dr.**
  **D-51373 Leverkusen (DE)**

(56) Entgegenhaltungen:
**US-A- 3 201 387** **US-A- 3 324 126**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 579 020 B1

## Beschreibung

5-(Trifluormethyl)-uracil ist ein wichtiges Zwischenprodukt zur Herstellung von Trifluridine (= 5'-Trifluoromethyl-2'-deoxyuridine = Trifluorothymidine = TFT), einem bekannten antiviralen Wirkstoff (s. Römpp's Chemie-Lexikon, 8.Auflage, 6.Band, S. 4350 (1988)):

Es ist bekannt, daß man 5-(Trifluormethyl)-uracil durch Umsetzung von Uracil-5-carbonsäure mit Schwefeltetrafluorid erhalten kann [siehe J. Pharm. Sci. 52 508, (1963)]. Dabei wird das sehr giftige Schwefeltetrafluorid in großem Überschuß und die schwierig zugängliche Uracil-5-carbonsäure als Ausgangsprodukt benötigt.

Aus der US-PS 3 201 387 ist die Verbindung 5-(Trifluormethyl)-uracil bekannt sowie ein Verfahren zu dessen Herstellung, bei dem man zunächst Trifluormethylacrylnitril mit Bromwasserstoff zu einem Bromamid umsetzt, das Bromamid mit überschüssigem Harnstoff zu einem N-substituierten Harnstoff kondensiert, dann diesen einer Hydrolyse und einer Cyclisierung unter Bildung eines Trifluormethyl-dihydro-dioxypyrimidins unterwirft, dieses Pyrimidin mit Brom behandelt und das so erhaltene Bromhydropyrimidin erhitzt.

Bei einem anderen bekannten Verfahren zur Herstellung von 5-(Trifluormethyl)-uracil geht man von Uracil-5-carbonsäure aus, das man mit einem Fluorierungsmittel umsetzt und aus dem Reaktionsgemisch fluoriertes Azin gewinnt (siehe US-PS 3 324 126).

Es wurde nun ein Verfahren zur Herstellung von 5-(Trifluormethyl)-uracil gefunden, das dadurch gekennzeichnet ist, daß man

a) Thymin unter milden Bedingungen chloriert, indem man Phosphoroxychlorid in Gegenwart eines tertiären Amins bei 20 bis 120°C auf Thymin einwirken läßt und so 2,4-Dichlor-5-methyl-pyrimidin erhält,

b) dieses unter drastischen Bedingungen zu 2,4-Dichlor-5-trichlormethylpyrimidin weiterchloriert, indem man bei Temperaturen von 180 bis 250°C mit elementarem Chlor oder Sulfurylchlorid unter UV-Bestrahlung und/oder in Gegenwart von Radikalbildnern und ohne Lösungsmittelzusatz chloriert,

c) dieses mit einem Fluorierungsmittel zu 2,4-fluorierten und/oder chlorierten 5-Trifluormethylpyrimidinen umsetzt und

d) diese einer Hydrolyse unterwirft.

Die vorliegende Erfindung betrifft auch die neue chemische Verbindung 2,4-Dichlor-5-trichlormethyl-pyrimidin, die nach Durchführung der Stufe b) isoliert werden kann und die neue chemische Verbindung 2,4-Difluor-5-trifluormethyl-pyrimidin, die nach Durchführung der Stufe c) isoliert werden kann.

Das erfindungsgemäße Verfahren kann durch folgendes Formelschema illustriert werden:

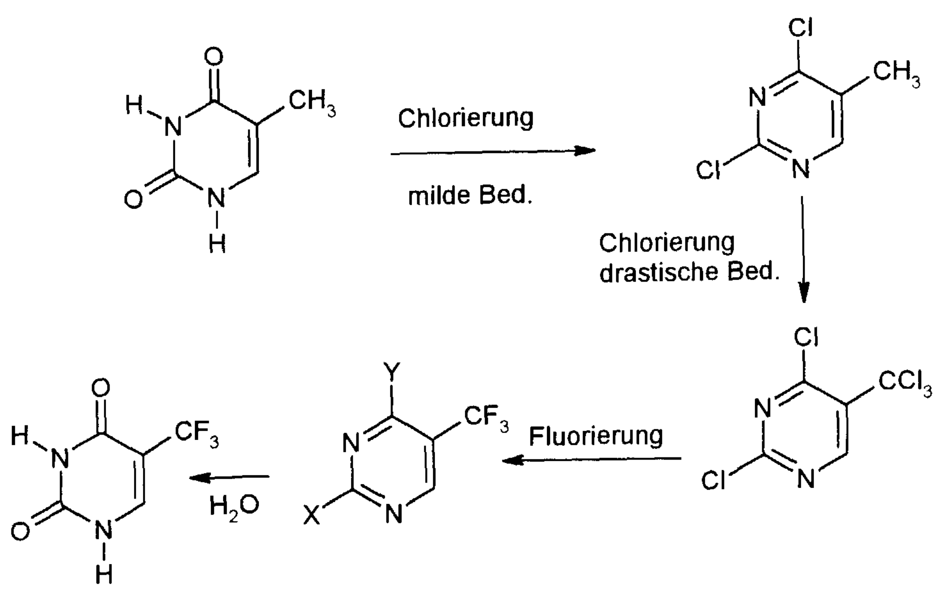


X und Y = Chlor oder Fluor

Das als Ausgangsprodukt benötigte Thymin (= 5-Methyluracil) ist im Handel erhältlich oder nach dem in J. Am.

Chem. Soc. 68, 912 (1946) beschriebenen Verfahren herstellbar.

In Stufe a) des erfindungsgemäßen Verfahrens kann man, bezogen auf 1 Mol Thymin, beispielsweise 3 bis 25 Mol Phosphoroxychlorid und 0,1 bis 1 Mol eines tertiären Amins, beispielsweise N,N-Dimethylanilin, einsetzen. Es ist im allgemeinen vorteilhaft, diese Reagenzien bei Raumtemperatur zusammenzugeben und die Reaktion bei Temperaturen zwischen 30°C und der Rückflußtemperatur, vorzugsweise bei am Rückfluß siedendem Phosphoroxychlorid, zu Ende zu führen.

Das dann vorliegende Reaktionsgemisch kann man beispielsweise aufarbeiten, indem man zunächst abkühlt, das überschüssige Phosphoroxychlorid entfernt, z.B. durch Destillation im Vakuum, anschließend den Rückstand mit Wasser verrührt, mit einem inerten organischen Lösungsmittel extrahiert und aus der organischen Phase das 2,4-Dichlor-5-methyl-pyrimidin isoliert. Nach der Abtrennung überschüssigen Phosphoroxychlorids kann man 2,4-Dichlor-5-methyl-pyrimidin auch durch Destillation isolieren. Die Ausbeute liegt in der Regel im Bereich 85 bis 95 %.

In Stufe b) des erfindungsgemäßen Verfahrens kann man als Radikalbildner beispielsweise Azoisobuttersäurenitril oder Peroxide zusetzen.

Die Einleitung des Chlorierungsmittels erfolgt zweckmäßigerweise so lange, bis man gaschromatografisch feststellt, daß alle vorhandenen Methyl-Wasserstoffatome durch Chlor substituiert worden sind. Das entstandene 2,4-Dichlor-5-trichlormethyl-pyrimidin ist eine neue chemische Verbindung und kann gewünschtenfalls durch Destillation im Vakuum gereinigt werden. Die Ausbeute in dieser Stufe liegt in der Regel bei 85 bis 95 %.

Die Stufe c) des erfindungsgemäßen Verfahrens kann man beispielsweise durchführen, indem man 2,4-Dichlor-5-trichlormethyl-pyrimidin, gegebenenfalls in Gegenwart einer katalytischen Menge von Antimonhalogeniden, mit überschüssigem Fluorwasserstoff unter Druck bei Temperaturen von 80 bis 180°C umsetzt und den entstehenden Chlorwasserstoff über einen Kühler mit Rückhalteventil entspannt. Wenn man die neue chemische Verbindung 2,4-Difluor-5-trifluormethyl-pyrimidin mit guten Ausbeuten und Selektivitäten erhalten will, so kann man mit und ohne Zusatz von Antimonhalogeniden arbeiten. Wenn man als Fluorierungsmittel Gemische von $SbF_3$ und $SbCl_5$ einsetzt, so werden im allgemeinen neben 2,4-Difluor- auch 2,4-Chlorfluor- und 2,4-Dichlor-5-trifluormethylpyrimidine in beachtlichen Anteilen erhalten. Dies ist für die Durchführung der Stufe d) des erfindungsgemäßen Verfahrens ohne besonderen Belang, denn alle diese 2,4-Dihalogen-5-trifluormethyl- pyrimidine lassen sich durch Hydrolyse in 5-Trifluormethyl-uracil überführen.

Man erhält auch dann im allgemeinen Gemische, die 2,4-Difluor-, 2,4-Chlorfluor- und 2,4-Dichlor-5-trifluormethyl-pyrimidine enthalten, wenn man als Fluorierungsmittel nur Fluorwasserstoff im Unterschuß einsetzt.

In Stufe c) kann man pro Mol 2,4-Dichlor-5-trichlormethyl-pyrimidin beispielsweise 5 bis 50 ml Antimonhalogenide, vorzugsweise Antimonpentachlorid oder Gemische aus Antimontrifluorid und Antimonpentachlorid einsetzen und, zusätzlich zu/oder anstatt der Antimonhalogenide, 60 bis 5 000 ml wasserfreien Fluorwasserstoff. Beim Einsatz von ca. 60 ml Fluorwasserstoff pro Mol des Pyrimidins wird im wesentlichen nur die Trichlormethylgruppe in eine Trifluormethylgruppe überführt. Beim Einsatz von Fluorwasserstoff z.B. im Bereich von ca. 65 bis 100 ml pro Mol des Pyrimidins wird im wesentlichen zusätzlich ein Kern-Chlor durch ein Fluoratom ersetzt, vorzugsweise das in 4-Position. Beim Einsatz von über 100 ml Fluorwasserstoff pro Mol des Pyrimidins werden im wesentlichen alle vorhandenen Chloratome durch Fluoratome ersetzt.

Bevorzugte Reaktionstemperaturen liegen im Bereich 120 bis 170°C. Der Druck kann beispielsweise 15 bis 40 bar, vorzugsweise 20 bis 30 bar, betragen. Die Aufarbeitung kann z.B. so erfolgen, daß man abkühlt, entspannt, überschüssige Fluorierungsmittel entfernt und den verbleibenden Rückstand destilliert. Die Ausbeute in dieser Stufe liegt in der Regel bei 70 bis 90 %.

Die Stufe d) des erfindungsgemäßen Verfahrens kann man beispielsweise durchführen, indem man die in Stufe c) erhaltenen 2,4-fluorierten und/oder chlorierten 5-Trifluormethyl-pyrimidine mit einem Überschuß an Wasser bei Raumtemperatur und/oder erhöhter Temperatur umsetzt. Bei Temperaturen im Bereich 20 bis 90°C ist die Verseifung häufig im Verlauf von 5 bis 24 Stunden abgeschlossen. Besonders vorteilhaft ist es, am Ende der Verseifungsreaktion die Temperatur anzuheben, z.B. bis zu kurzem Sieden, und dann das Gemisch heiß zu filtrieren. Beim Abkühlen des Filtrates kristallisiert dann 5-Trifluormethyl-uracil aus. Bezogen auf 1 g 2,4-fluorierte und/oder chlorierte 5-Trifluormethyl-pyrimidine kann man z.B. 3 bis 20 ml Wasser einsetzen. Die Ausbeute in dieser Stufe liegt in der Regel bei 85 bis 95 %.

Es ist vorteilhaft, dem Überschuß an Wasser Kaliumfluorid und/oder Natriumfluorid zuzusetzen, beispielsweise 0,5 bis 2 Mol der Fluoride pro Mol 2,4-dihalogeniertem 5-Trifluormethyl-pyrimidin. Das erfindungsgemäße Verfahren hat eine Reihe von Vorteilen: Es kann mit gut zugänglichen Ausgangsprodukten und einfachen Reagenzien (z.B. $POCl_3$, $Cl_2$, HF und $H_2O$), durchgeführt werden, es liefert in guten Ausbeuten und mit wenig Abfallstoffen 5-Trifluormethyl-uracil und es ist in technischem Maßstab gut durchführbar.

Überraschend ist, daß die Chlorierung in der Stufe b) in guter Ausbeute zur Trichlormethylverbindung führt und praktisch kaum $CHCl_2$- und $CH_2Cl$-Derivate und praktisch keine Zersetzungen beobachtet werden. Ferner ist überraschend, daß in Stufe c) in einem Schritt bis zu 5 Fluoratome eingeführt werden können, die sich dann sowohl am aromatischen Kern, als auch in der Methyl-Seitenkette befinden. Schließlich ist überraschend, daß in Stufe d) nur die

Kern-Fluor- oder Kern-Chloratome verseift werden und die $CF_3$-Gruppe intakt bleibt. Es hätte erwartet werden können, daß auch diese unter Bildung einer COOH-Gruppe verseift wird.

Beispiele

Beispiel 1 (Herstellung von 2,4-Dichlor-5-methyl-pyrimidin)

Zu 3 067 g Phosphoroxychlorid wurden unter Kühlung langsam 48 g Dimethylanilin getropft und 5 Minuten bei 25°C nachgerührt. Dann wurden langsam 252 g Thymin (= 5-Methyl-uracil) bei 25°C zugegeben und anschließend 20 Stunden unter Rückfluß nachgerührt. Nach dem Abkühlen wurde noch vorhandenes überschüssiges Phosphoroxychlorid bei 30 bis 35°C im Wasserstrahlvakuum abdestilliert und der verbleibende Rückstand im Vakuum destilliert. Es wurden 301 g (= 92 % d. Th.) 2,4-Dichlor-5-methyl-pyrimidin mit einem Siedepunkt von 110° C bei einem Druck von 16 mbar erhalten.

Beispiel 2 (Herstellung von 2,4-Dichlor-5-trichlormethyl-pyrimidin)

In einem Dreihalskolben mit Rückflußkühler wurden 257 g 2,4-Dichlor-5-methyl-pyrimidin vorgelegt und dann unter Bestrahlung mit einer UV-Lampe und allmählicher Temperatursteigerung bis auf 230°C so lange Chlor eingeleitet (ca. 35 Stunden) bis gemäß gaschromatografischer Analyse das $CCl_3$-Produkt entstanden war. Das so erhaltene 2,4-Dichlor-5-trichlormethyl-pyrimidin wurde durch Destillation isoliert. Es wies einen Siedepunkt von 80 bis 82° C bei 0,08 mbar und einen Brechungsindex $n_D^{20}$ von 1,5903 auf. Es wurde in einer Ausbeute von 387 g (= 92 % d. Th.) erhalten.

Das $^1$H-NMR-Spektrum (200 MHz, $CDCl_3$) wies ein charakteristisches Signal bei $\delta$ = 9,3 ppm auf. Das Massenspektrum (EI, 70eV) wies charakteristische Banden bei m/z = 266 (6 %), 231 (100 %), 195 (10 %), 161 (4 %), 141 (26 %) und 107 (18 %) auf.

Beispiel 3 (Herstellung von 2,4-Difluor-5-trifluormethyl-pyrimidin)

In einem Edelstahl-Rührautoklaven wurden 400 g 2,4-Dichlor-5-trichlormethyl-pyrimidin, 30 ml Antimonpentachlorid und 1700 ml wasserfreie Flußsäure vorgelegt und 4 Stunden bei 150°C und einem Druck von 30 bar unter Stickstoff gerührt. Der entstehende Chlorwasserstoff wurde über einen Kühler kontinuierlich entspannt. Nach Ende der Chlorwasserstoff-Entwicklung wurde abgekühlt, entspannt, die überschüssige Flußsäure abdestilliert und das erhaltene 2,4-Difluor-5-trifluormethyl-pyrimidin mit einem Siedepunkt von 105°C bei Normaldruck abdestilliert. Die Ausbeute betrug 204 g (= 74 % d. Th.).

Das $^1$H-NMR-Spektrum (200 MHz, $CDCl_3$) wies ein charakteristisches Signal bei $\delta$ = 9,02 ppm auf. Das $^{19}$F-NMR-Spektrum (188 MHz, $CDCl_3$) wies charakteristische Linien bei $\delta$ = -36,65 ppm, -52,85 ppm und -62,02 ppm auf. Das Massenspektrum (EI, 70eV) wies charakteristische Absorptionen bei m/z = 184 (85 %), 165 (80 %), 138 (15 %), 119 (15 X), 93 (55 %), 69 (70 %) und 31 (100 %) auf.

Beispiel 4 (Herstellung von 5-Trifluormethyl-uracil)

18,4 g 2,4-Difluor-5-trifluormethyl-pyrimidin wurden mit einer Lösung von 11,3 g Kaliumfluorid in 100 ml Wasser versetzt und 15 Stunden bei 25 bis 30°C gerührt. Dann wurde kurz aufgekocht, heiß filtriert, das Filtrat eingeengt und abkühlen gelassen. Dabei kristallisierte 5-Trifluormethyl-uracil mit einem Schmelzpunkt von 249 bis 251°C aus. Die Ausbeute betrug 16,4 g (= 91 % d.Th.).

Folgende charakteristische Daten wurden durch NMR-Spektroskopie erhalten: $^1$H-NMR (200 MHz, Dimethylsulfoxid): $\delta$ = 11,5 und 8,0; $^{19}$F-NMR (188 MHz, Dimethylsulfoxid): $\delta$ = -61,1.

Beispiel 5 (Herstellung von 2,4-Difluor-5-trifluormethyl-pyrimidin)

In einem Edelstahl-Rührautoklaven wurden bei 0°C 500 g 2,4-Dichlor-5-trichlormethyl-pyrimidin und 600 ml wasserfreie Flußsäure vorgelegt. Die Apparatur wurde verschlossen, 10 bar Stickstoff aufgedrückt und auf 142°C erhitzt. Der entstandene Chlorwasserstoff wurde über einen Kühler kontinuierlich entspannt. Die Reaktionszeit betrug 4 Stunden. Danach wurde abgekühlt, entspannt, zunächst die überschüssige Flußsäure und dann das Produkt durch Destillation abgetrennt. Es wurden 311 g (= 90 % der Theorie) 2,4-Difluor-5-trifluormethyl-pyrimidin mit einem Siedepunkt von 105°C bei Normaldruck erhalten.

<u>Beispiel 6</u> (Herstellung eines 2,4-Dihalogen-5-trifluormethyl-pyrimidin-Gemisches)

In einer Rührapparatur wurden 27 g 2,4-Dichlor-5-trichlormethyl-pyrimidin, 50 g Antimontrifluorid und 5 g Antimonpentachlorid vorgelegt und bei 150 bis 160°C 4 Stunden gerührt. Die im Reaktionsgemisch enthaltenen Trifluormethylpyrimidine wurden aus dem Reaktionsgemisch durch Destillation im Vakuum abgetrennt. Es wurden 10 g eines Produktgemisches erhalten, das gemäß $^1$H-NMR-spektroskopischer Analyse zu 44 Gew.-% aus 2,4-Difluor-5-trifluormethyl-pyrimidin, zu 36 Gew.-% aus 2-Chlor-4-fluor-5-trifluormethyl-pyrimidin und zu 20 Gew.-% aus 2,4-Dichlor-5-trifluormethyl-pyrimidin bestand.

<u>Beispiel 7</u> (Herstellung von 5-Trifluormethyl-uracil)

510 g Kaliumfluorid wurden in 4.900 ml Wasser vorgelegt und mit 1.620 g 2,4-Difluor-5-trifluormethyl-pyrimidin versetzt. Dann wurde 5 Stunden bei 50°C gerührt. Anschließend wurde das auskristallisierte 5-Trifluormethyl-uracil abgesaugt. Es wurden 1.442 g (= 91 % der Theorie) mit einem Schmelzpunkt von 251 bis 252°C erhalten.

**Patentansprüche**

1. Verfahren zur Herstellung von 5-(Trifluormethyl)-uracil, dadurch gekennzeichnet, daß man

   a) Thymin unter milden Bedingungen chloriert, indem man Phosphoroxychlorid in Gegenwart eines tertiären Amins bei 20 bis 120°C auf Thymin einwirken läßt und so 2,4-Dichlor-5-methyl-pyrimidin erhält,

   b) dieses unter drastischen Bedingungen zu 2,4-Dichlor-5-trichlormethylpyrimidin weiterchloriert, indem man bei Temperaturen von 180 bis 250°C mit elementarem Chlor oder Sulfurylchlorid unter UV-Bestrahlung und/oder in Gegenwart von Radikalbildnern und ohne Lösungsmittelzusatz chloriert,

   c) das so erhaltene 2,4-Dichlor-5-trichlormethyl-pyrimidin mit einem Fluorierungsmittel zu 2,4-fluorierten und/oder chlorierten 5-Trifluormethylpyrimidinen umsetzt und

   d) diese einer Hydrolyse unterwirft.

2. Verfahren nach Anspruch 1, dadurch gekennzeichnet, daß man in Stufe c) bei 80 bis 180°C mit einer katalytischen Menge von Antimonhalogeniden und überschüssigem Fluorwasserstoff oder Gemischen aus Antimontrifluorid und Antimonpentachlorid oder nur mit Fluorwasserstoff fluoriert.

3. Verfahren nach Ansprüchen 1 und 2, dadurch gekennzeichnet, daß man die Stufe d) bei 20 bis 90°C durchführt.

4. Verfahren nach Ansprüchen 1 bis 3, dadurch gekennzeichnet, daß man die Hydrolyse mit einem Überschuß an Wasser und in Gegenwart von Kaliumfluorid und/oder Natriumfluorid durchführt.

5. Die Verbindung 2,4-Dichlor-5 -trichlormethylpyrimidin.

6. Die Verbindung 2,4-Difluor-5-trifluormethylpyrimidin.

**Claims**

1. Process for the preparation of 5-(trifluoromethyl)uracil, characterised in that

   a) thymine is chlorinated under mild conditions by reacting phosphorus oxychloride with thymine in the presence of a tertiary amine at 20 to 120°C to give 2,4-dichloro-5-methyl-pyrimidine,

   b) this is further chlorinated under drastic conditions by carrying out chlorination at temperatures of 180 to 250°C with elemental chlorine or sulphuryl chloride under UV irradiation and/or in the presence of radical-forming agents and without the addition of a solvent to give 2,4-dichloro-5-trichloromethyl- pyrimidine,

   c) the 2,4-dichloro-5-trichloromethyl-pyrimidine obtained in this manner is reacted with a fluorinating agent to

give 2,4-fluorinated and/or chlorinated 5-trifluoro-methylpyrimidines, and

d) these are subjected to hydrolysis.

**2.** Process according to Claim 1, characterised in that, in step c), fluorination is carried out at 80 to 180°C with a catalytic amount of antimony halides and excess hydrogen fluoride, or mixtures of antimony trifluoride and antimony pentachloride, or only hydrogen fluoride.

**3.** Process according to Claims 1 and 2, characterised in that step d) is carried out at 20 to 90°C.

**4.** Process according to Claims 1 to 3, characterised in that the hydrolysis is carried out with excess water and in the presence of potassium fluoride and/or sodium fluoride.

**5.** The compound 2,4-dichloro-5-trichloromethylpyrimidine.

**6.** The compound 2,4-difluoro-5-trifluoromethylpyrimidine.

## Revendications

**1.** Procédé pour la préparation du 5-(trifluorométhyl)uracile, caractérisé en ce que

a) on soumet de la thymine à une chloration dans des conditions douces en laissant agir de l'oxychlorure de phosphore sur la thymine en présence d'une amine tertiaire, à une température de 20 à 120°C, et on obtient ainsi la 2,4-dichloro-5-méthyl-pyrimidine,

b) on soumet cette dernière à une chloration ultérieure dans des conditions drastiques pour obtenir la 2,4-dichloro-5-trichlorométhylpyrimidine en procédant à la chloration à des températures de 180 à 250°C avec du chlore élémentaire ou du chlorure de sulfuryle sous un rayonnement UV et/ou en présence de formateurs de radicaux et sans addition de solvants,

c) on fait réagir la 2,4-dichloro-5-trichlorométhyl-pyrimidine ainsi obtenue avec un agent de fluoration pour obtenir des 5-trifluorométhyl-pyrimidines 2,4-fluorées et/ou chlorées, et

d) on soumet ces dernières à une hydrolyse.

**2.** Procédé selon la revendication 1, caractérisé en ce que, à l'étape c), on effectue la fluoration à une température de 80 à 180°C avec une quantité catalytique d'halogénures d'antimoine et de l'acide fluorhydrique en excès ou encore de mélanges de trifluorure d'antimoine et de pentachlorure d'antimoine ou encore uniquement avec de l'acide fluorhydrique.

**3.** Procédé selon les revendications 1 et 2, caractérisé en ce qu'on effectue l'étape d) à une température de 20 à 90°C.

**4.** Procédé selon les revendications 1 à 3, caractérisé en ce qu'on effectue l'hydrolyse avec un excès d'eau et en présence de fluorure de potassium et/ou de fluorure de sodium.

**5.** Le composé 2,4-dichloro-5-trichlorométhylpyrimidine.

**6.** Le composé 2,4-difluoro-5-trifluorométhylpyrimidine.